(19) Europäisches Patentamt
European Patent Office
Office européen des brevets

(11) EP 2 547 783 B1

(12) FASCICULE DE BREVET EUROPEEN

(45) Date de publication et mention
de la délivrance du brevet:
**11.11.2015 Bulletin 2015/46**

(51) Int Cl.:
***C12Q 1/06*** (2006.01)

(21) Numéro de dépôt: **11715935.0**

(22) Date de dépôt: **17.03.2011**

(86) Numéro de dépôt international:
**PCT/FR2011/050543**

(87) Numéro de publication internationale:
**WO 2011/114062 (22.09.2011 Gazette 2011/38)**

(54) **PROCEDE D'ESTIMATION DU NOMBRE DE MICROORGANISMES DANS UN ECHANTILLON ET DISPOSITIF POUR METTRE EN OEUVRE LEDIT PROCEDE**

VERFAHREN ZUR BERECHNUNG DER ANZAHL AN MIKROORGANISMEN IN EINER PROBE UND VORRICHTUNG ZUR UMSETZUNG DES VERFAHRENS

METHOD FOR ESTIMATING THE NUMBER OF MICROORGANISMS IN A SAMPLE AND DEVICE FOR IMPLEMENTING SAID METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.03.2010 FR 1052010**

(43) Date de publication de la demande:
**23.01.2013 Bulletin 2013/04**

(73) Titulaire: **BIOMERIEUX**
**69280 Marcy-L'Etoile (FR)**

(72) Inventeurs:
• **DEVULDER, Grégory**
  **F-69510 Thurins (FR)**
• **ARTHAUD, Catherine**
  **F-69100 Villeurbanne (FR)**
• **COTTE-PATTAT, Pierre-Jean**
  **F-01150 Lagnieu (FR)**
• **RAYMOND, Jean-Claude**
  **F-69690 Bessenay (FR)**

(74) Mandataire: **Cabinet Laurent & Charras**
**Le Contemporain**
**50 Chemin de la Bruyère**
**69574 Dardilly Cedex (FR)**

(56) Documents cités:
**FR-A1- 2 782 729      US-A1- 2008 113 404**

• **ZHENG LU ET AL: "Application of predictive models to estimate Listeria monocytogenes growth on frankfurters treated with organic acid salts", JOURNAL OF FOOD PROTECTION, INTERNATIONAL ASSOCIATION FOR FOOD PROTECTION, US, vol. 68, no. 11, 1 janvier 2005 (2005-01-01), pages 2326-2332, XP009141121, ISSN: 0362-028X**
• **JUNEJA V K ET AL: "Mathematical modeling of growth of Salmonella in raw ground beef under isothermal conditions from 10 to 45 <o>C", INTERNATIONAL JOURNAL OF FOOD MICROBIOLOGY, ELSEVIER SCIENCE PUBLISHERS, AMSTERDAM, NL, vol. 131, no. 2-3, 31 mai 2009 (2009-05-31), pages 106-111, XP026042768, ISSN: 0168-1605, DOI: DOI: 10.1016/J.IJFOODMICRO.2009.01.034 [extrait le 2009-02-05]**

**Description**

**DOMAINE DE L'INVENTION**

L'invention a trait au domaine du dénombrement de microorganismes, tels que des bactéries et/ou des champignons, présents dans un échantillon biologique.

**ETAT DE LA TECHNIQUE**

Le dénombrement de microorganismes présents dans un échantillon biologique revêt une importance particulière dans nombre de domaines, et plus particulièrement dans celui de la sécurité alimentaire.

La mise sur le marché d'un produit alimentaire est soumise, pour des raisons évidentes de sécurité, à une règlementation stricte concernant la charge bactérienne maximale acceptable que doit contenir le produit. Des contrôles de qualité sont ainsi mis en oeuvre pour savoir si le produit alimentaire en question est propre à la consommation et peut donc être commercialisé.

Les contrôles de qualité usuels consistent essentiellement en une première étape de préparation d'un échantillon biologique à partir du produit à tester, suivie d'une deuxième étape d'énumération des microorganismes présents dans cet échantillon après incubation. Dès lors que le nombre de microorganismes compté excède un seuil prédéterminé, le produit testé est alors considéré comme impropre, et donc non commercialisé, ou bien, le cas échéant, retiré du marché s'il était déjà commercialisé.

Toutefois, quelle que soit la méthode de comptage employée, il convient de soumettre l'échantillon à une incubation plus ou moins longue en fonction du produit testé et du milieu de culture utilisé. En effet, selon la méthode mise en oeuvre, le temps d'incubation nécessaire pour obtenir un dénombrement de microorganismes peut varier de 24h jusqu'à 5 jours. Dans le cas du dénombrement de la flore totale d'un échantillon, le temps d'incubation est au minimum de 48 h à 35°C, voire de 72h à 30°C. Ainsi, pour un produit alimentaire cru, seul le dénombrement après un minimum de 40 heures d'incubation, et plus particulièrement au moins 48 heures dans le cas d'une viande crue, est considéré comme fiable.

Toutefois, une quarantaine d'heures est une durée excessivement longue au regard de la durée de vie de certains produits. En effet par exemple, la durée de vie d'une viande crue n'excède pas quelques jours. Alors même que le processus de mise sur le marché est généralement déjà engagé pour qu'un produit se retrouve au plus tôt commercialisé dès que les tests de qualité sont positifs, un résultat finalement négatif interdit cette commercialisation, ce qui entraine une perte économique importante.

On conçoit donc aisément l'utilité d'un procédé de comptage qui raccourcit grandement le temps d'incubation tout en produisant un comptage sensiblement aussi fiable qui celui obtenu après un temps d'incubation important.

**EXPOSE DE L'INVENTION**

Le but de l'invention est de proposer un procédé et un dispositif qui obtiennent un tel résultat.

A cet effet, l'invention a pour objet un procédé de dénombrement de microorganismes présents dans un échantillon biologique en contact avec un milieu de culture adapté à la croissance desdits microorganismes.

Selon l'invention, le procédé comprend les étapes consistant à :

- déterminer à un stade antérieur le nombre $N_{SA}$ de microorganismes présents dans l'échantillon
- calculer le nombre $N_{SU}$ de microorganismes présents à un stade ultérieur en fonction du nombre $N_{SA}$, le calcul se fondant sur un modèle de croissance des microorganismes dans le milieu de culture selon la relation :

$$\log(N_{SU}) = \alpha \times \log(N_{SA}) - \beta \times \log(C_{SA}) + \gamma$$

où log est le logarithme décimal, $N_{SU}$ est le nombre de microorganismes calculé, $N_{SA}$ est le nombre de microorganismes au stade antérieur, $C_{SA}$ le nombre de microorganismes au stade antérieur divisé par le volume de l'échantillon, et $\alpha, \beta$ et $\gamma$ sont des paramètres prédéterminés dépendants des microorganismes, du milieu de culture et de la durée séparant le stade ultérieur du stade antérieur, $\alpha$ et $\beta$ étant positifs.

Selon l'invention, il convient de comprendre l'expression « nombre de microorganismes » au sens large. Il peut ainsi s'agir du nombre réel de microorganismes ou du nombre de colonies de microorganismes (par exemple en Unité Formant Colonie).

Par « se fondant sur un modèle», il faut ici comprendre tout calcul utilisant ledit modèle, que ce soit directement sous la forme mathématique exposée ci-dessus ou sous une forme mathématique équivalente.

En d'autres termes, grâce au modèle utilisé, le nombre de microorganismes, bactéries et/ou champignons, présents dans l'échantillon biologique après une durée d'incubation normalement nécessaire, est extrapolé de manière fiable à partir d'un nombre de microorganismes compté après une durée d'incubation réelle plus courte. Il est ainsi obtenu un gain de temps égal à la différence entre ces durées d'incubation normalement nécessaire et réelle.

Selon un mode de réalisation de l'invention, le procédé comporte la détermination du nombre de microorganismes au stade antérieur, ladite estimation au stade antérieure comportant :

- la division de l'échantillon entre plusieurs ensembles identiques de volume identique ou d'au moins deux volumes différents, selon un rapport de proportionnalité prédéterminé ;
- l'application de conditions de culture auxdits volumes, notamment une incubation à une température prédéterminée pendant une durée prédéterminée, afin de faire croitre le nombre de microorganismes dans ces volumes ;
- la détection de la présence ou de l'absence de microorganismes dans les volumes après l'application des conditions de culture ; et
- le calcul du nombre de microorganismes dans l'échantillon par la méthode du Nombre le Plus Probable (NPP).

Selon un mode de réalisation de l'invention, le procédé comporte une étape de préparation de l'échantillon biologique à partir d'une portion de produit alimentaire.

Selon un mode particulier de réalisation de l'invention dans lequel on dénombre la flore totale dans une produit frais avec un milieu de culture adéquate au moyen de l'instrument TEMPO® commercialisé par la demanderesse, le stade antérieur correspond à environ 24 heures après la préparation de l'échantillon, et le stade ultérieur correspond à environ 48 heures après la préparation de l'échantillon.

L'invention a également pour objet un dispositif pour le dénombrement de microorganismes présents dans un échantillon biologique en contact avec un milieu de culture adapté à la croissance desdits microorganismes, comportant des moyens formant réceptacle pour échantillon, et des moyens de mesure ou d'estimation du nombre de microorganismes présents dans un échantillon disposé dans le réceptacle pour échantillon.

Selon l'invention, le dispositif comporte en outre des moyens de calcul du nombre de microorganismes présents dans l'échantillon en fonction du nombre de microorganismes mesuré ou estimé par les moyens de mesure ou d'estimation, en se fondant sur un modèle de croissance des microorganismes selon la relation :

$$\log(N_{SU}) = \alpha \times \log(N_{SA}) - \beta \times \log(C_{SA}) + \gamma$$

où log est le logarithme décimal, $N_{SU}$ est le nombre de microorganismes calculé au stade ultérieur, $N_{SA}$ est le nombre de microorganismes déterminé au stade antérieur, $C_{SA}$ le nombre de microorganismes au stade antérieur divisé par le volume de l'échantillon, et $\alpha$, $\beta$ et $\gamma$ sont des paramètres prédéterminés dépendants de l'échantillon, du milieu de culture et de la durée séparant le stade ultérieur du stade antérieur, $\alpha$ et $\beta$ étant positifs.

L'invention a également pour objet un produit programme d'ordinateur comprenant des instructions, stockées sur un support d'enregistrement lisible par ordinateur, pour mettre en oeuvre le procédé du type précité lorsque le programme est exécuté par un ordinateur.

**BREVE DESCRIPTION DES FIGURES**

L'invention sera mieux comprise à la lecture de la description qui va suivre, donnée uniquement à titre d'exemple, et faite en relation avec les dessins annexés, dans lesquels :

- la figure 1 est un organigramme d'un procédé selon l'invention ; et
- la figure 2 est un tracé d'expériences avec pour abscisses des comptages de colonies effectués après 48 heures d'incubation de différents échantillons biologiques issus de viandes crues, et pour ordonnées le comptage estimé d'après l'invention.

**DESCRIPTION DETAILLEE DE L'INVENTION**

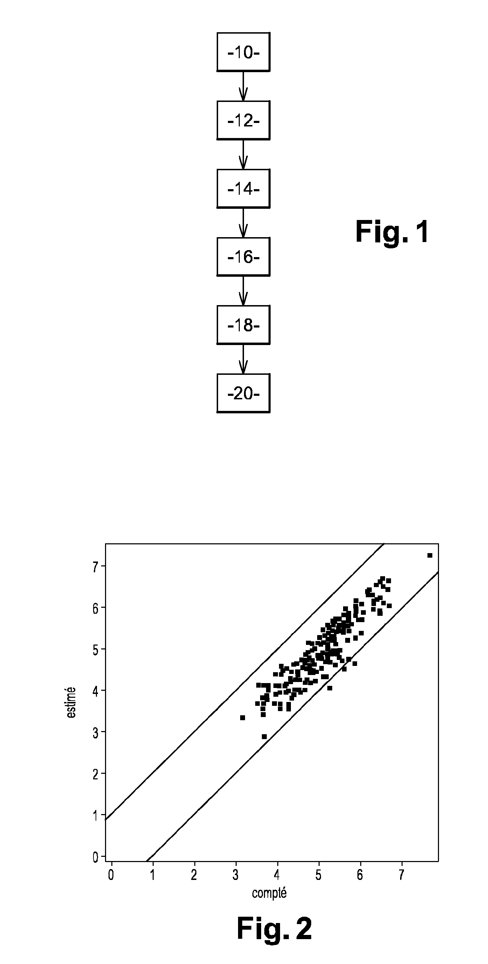

En se référant à la figure 1, un procédé selon l'invention comporte une première étape 10 de préparation d'un échantillon biologique à partir d'un produit à tester.

Par exemple, pour dénombrer les microorganismes présents dans une viande crue, essentiellement des bactéries, une masse prédéterminée de cette viande est disposée dans un sac Stomacher avec un volume d'eau peptonnée. Une homogénéisation de l'ensemble est réalisée dans le sac pendant plusieurs minutes. Le liquide obtenu subit alors une dilution supplémentaire en transférant un volume du liquide contenu dans le sac Stomacher dans un flacon contenant un volume de milieu de culture. Un échantillon biologique liquide est alors obtenu, cet échantillon étant soumis au comptage selon l'invention.

De façon classique, le milieu de culture est choisi en fonction du type de microorganismes dont on cherche à savoir s'ils sont ou non présents dans l'échantillon biologique, et dont on cherche à connaître le nombre après une durée donnée d'incubation. Le milieu de culture est à large spectre, c'est-à-dire adapté à la croissance d'un grand nombre de microorganismes de types différents susceptibles d'être présents dans le produit testé, ou est sélectionné pour convenir à la croissance d'un nombre restreint de types de microorganismes, voire pour un seul type de microorganisme. Le choix du milieu de culture dépend de l'application visée, comme cela est connu en soi de l'état de la technique. Par exemple, dans le cas d'une viande crue, un milieu de culture à large spectre est privilégié.

Une fois l'échantillon biologique obtenu, celui-ci est alors préparé, en 12, pour être soumis à un comptage d'Unités Formant Colonie par gramme (UFC/g) en utilisant la méthode du nombre le plus probable (ou méthode « NPP »).

Plus particulièrement, l'échantillon est distribué entre plusieurs ensembles identiques de N cavités transparentes, isolées, de volumes proportionnels, et formées dans une carte d'analyse. Classiquement, chaque ensemble présente trois cavités, le volume de la grande cavité étant 10 fois supérieur au volume de la cavité moyenne, elle-même de volume 10 fois supérieur au volume de la petite cavité. De préférence, la distribution de l'échantillon biologique est réalisée au moyen d'une carte d'analyse telle que décrite dans le document EP 1 105 457 qui a l'avantage d'automatiser de façon fiable la distribution de l'échantillon entre plusieurs cavités transparentes, tout en isolant celles-ci les unes par rapport aux autres.

La carte d'analyse est alors soumise, en 14, pendant une durée prédéterminée à des conditions favorables pour la croissance des microorganismes que l'on souhaite dénombrer, notamment une incubation à une température donnée, par exemple entre 30°C et 35°C pour des microorganismes contenus dans des produits alimentaires.

Une fois la durée d'incubation terminée, le procédé se poursuit, en 16, par la détection optique de la présence ou l'absence de microorganismes dans chacune des cavités de la carte d'analyse. En fonction du milieu de culture choisi, il est en effet possible de faire croître certains microorganismes. Or, la croissance des microorganismes a pour effet de modifier la densité optique ou la coloration du contenu d'une cavité (par exemple dans le cas d'utilisation de marqueurs fluorescents ou chromogènes dans le milieu de culture), comme cela est connu en soi de l'état de la technique. Une modification mesurable des propriétés optiques d'une cavité signifie ainsi un développement microbien dans celle-ci, ou sinon une absence de développement microbien.

De préférence, dans le cas de l'analyse d'un produit frais, une incubation d'une durée comprise entre 20 et 40 heures permet d'obtenir une estimation fiable du nombre de microorganismes entre 40 et 76 heures, au regard de la détection optique mise en oeuvre et de la méthode NPP. En particulier, dans le cas d'une analyse de viande crue au moyen du système de détection TEMPO® TVC vendu par la société bioMérieux, on peut obtenir, entre 20 et 36 heures, une estimation du nombre de microorganismes entre 40 et 48 heures.

Une fois déterminée la présence ou l'absence de développement de microorganismes dans chacune des cavités de la carte d'analyse, un comptage d'unités formant colonie par gramme est réalisé, en **18**, en utilisant la méthode NPP, préférentiellement celle de J.C. De Man. On se référera par exemple au document EP 1 105 457 pour plus de détails sur cette méthode.

Il est ainsi obtenu un nombre d'unités formant colonie par gramme de produit testé, et donc le nombre d'unités formant colonie présentes dans l'échantillon biologique. Ce dernier nombre diffère du premier nombre par un facteur de proportionnalité tenant compte des diverses dilutions réalisées pour préparer l'échantillon et des volumes d'échantillon prélevé initialement.

Le procédé se poursuit alors, en **20**, en estimant par calcul le nombre d'unités formant colonie qui seraient obtenues par une incubation normalement nécessaire plus longue de l'échantillon biologique, par exemple 48 heures pour les viandes crues, en fonction du nombre d'unités formant colonie présentes dans l'échantillon compté suite à l'incubation réelle, selon la relation :

$$\log(N_{SU}) = \alpha \times \log(N_{SA}) - \beta \times \log(C_{SA}) + \gamma$$

où log est le logarithme décimal, $N_{SU}$ est le nombre estimé pour l'incubation normalement nécessaire, $N_{SA}$ est le nombre d'unités formant colonie dans l'échantillon compté suite à l'incubation réelle, $C_{SA}$ est ce même nombre divisé par le volume de l'échantillon biologique, et $\alpha$, $\beta$ et $\gamma$ sont des paramètres positifs prédéterminés dépendants des microorganismes, du milieu de culture et de la durée séparant l'incubation normalement nécessaire de l'incubation réelle.

On notera ici que ce calcul peut être effectué alors même que l'incubation se poursuit, la carte d'analyse étant par exemple disposée dans une chambre d'incubation pourvue de moyens optiques de détection raccordés à une unité de traitement d'informations mettant en oeuvre la méthode NPP et le comptage selon l'invention.

Les paramètres α, β et γ sont déterminés a priori, par exemple selon la procédure suivante :

a) préparer un nombre statistiquement significatif d'échantillons biologiques à partir d'un type de produit à tester à un stade ultérieur (par exemple de la viande crue) à différents stades de contamination ;

b) incuber lesdits échantillons pendant la durée d'incubation qui sera utilisée pour le comptage sur lequel se base l'estimation, et compter le nombre de microorganismes dans lesdits échantillons après incubation ;

c) poursuivre l'incubation des échantillons jusqu'au stade ultérieur désiré pour l'estimation, et compter le nombre de microorganismes dans les échantillons après cette incubation supplémentaire ; et

d) identifier par une méthode statistique les paramètres du modèle, par exemple au moyen d'une ajustement dans l'espace des logarithmes.

Le procédé est préférentiellement mis en oeuvre au moyen du système de diagnostic TEMPO®, ce système de diagnostic utilisant les cartes d'analyse décrites au document EP 1 105 457.

Le système TEMPO® comprend une unité de traitement d'informations, par exemple un microcontrôleur, raccordée au circuit de détection optique pour mettre en oeuvre le comptage selon la méthode NPP à partir des résultats de la détection. Selon l'invention, ce dispositif est modifié pour que l'unité de traitement d'informations soit en outre apte à mettre en oeuvre l'estimation selon l'invention.

De manière avantageuse, l'unité de traitement d'informations mémorise différents ensembles de paramètres α, β et γ, et l'utilisateur peut sélectionner, à l'aide d'une interface raccordée à l'unité de traitement d'informations, l'ensemble approprié pour l'échantillon biologique dans la carte d'analyse et la durée d'incubation pour lequel l'estimation est réalisée. Le cas échéant, la durée de l'incubation réelle est réglable et l'ensemble de paramètres également sélectionné en fonction de cette durée.

Un exemple de dénombrement de microorganismes dans de la viande crue mis en oeuvre au moyen du système TEMPO® est décrit ci-après.

Un échantillon biologique est tout d'abord préparé. Une dilution primaire au dixième est préparée en mélangeant 10g de l'échantillon avec un volume de 90 millilitres (mL) de diluant primaire, telle que de l'eau peptonée, dans un sac Stomacher. Une homogénéisation de l'ensemble est réalisée dans le sac pendant 1 à 2 minutes.

Ensuite, des cartes TEMPO® sont préparées avec le milieu de culture.

Le milieu de culture TEMPO® TVC est reconstitué en distribuant 3 mL de diluant secondaire, par exemple de l'eau distillée, dans un flacon TEMPO® TVC.

A l'aide d'une pipette stérile, 1 mL est alors prélevé dans le compartiment filtré du sac Stomacher et est transféré dans le flacon TEMPO® TVC contenant le milieu de culture reconstitué, afin d'ensemencer ce dernier. Le tout est homogénéisé à l'aide d'un agitateur de type vortex durant environ 3 secondes. Les 4 mL de milieu ensemencé obtenus correspondent donc à une dilution au 1/40 de l'échantillon.

Le flacon contenant le milieu ensemencé est placé sur un portoir de remplissage. La carte TEMPO® correspondante est placée sur le portoir en face du flacon, avec le tube de transfert de la carte plongé dans le flacon.

Le portoir contenant les flacons et les cartes, en connexion fluide, est placé dans le TEMPO® Filler. Le cycle de remplissage est alors lancé. Le milieu ensemencé est aspiré en totalité dans la carte. Après remplissage des cartes, ces dernières sont isolées de l'environnement extérieur par coupure et scellage des tubes de transfert.

Le portoir de remplissage est sorti du TEMPO® Filler. Les cartes sont retirées du portoir et transférées sur des portoirs d'incubation.

Les cartes sont alors incubées à température de 35°C pour l'énumération de la flore totale.

Le procédé se poursuit alors par la lecture des cartes.

Après 24h d'incubation, les portoirs de lecture sont placés dans le lecteur TEMPO® Reader. La lecture préliminaire est alors automatiquement réalisée. Pour chacun des puits de la carte, le système défini la positivité, puis transforme cela en une combinaison NPP.

A partir de la combinaison obtenue et du facteur de dilution appliqué, le système calcule automatiquement la concentration en micro-organismes dans l'échantillon. Le modèle mathématique défini est alors appliqué afin d'obtenir le résultat final estimé.

Il a été estimé le nombre de microorganismes, qui seraient présents dans les échantillons après 48 heures d'incubation à 35°C à partir d'un comptage réalisé après 24 heures d'incubation à 35°C, à l'aide du modèle :

$$\log(N_{SU}) = 1.072 \times \log(N_{SA}) - 0{,}232 \times \log(C_{SA}) + 0.94$$

Les résultats d'expérience sont présentés sur le tracé de la figure 2. Les abscisses de cette figure représentent le nombre compté de microorganismes dans les échantillons après une incubation réelle de 48 heures à 35°C. Les ordonnées représentent quant à elles les nombres estimés correspondants de microorganismes dans les échantillons sur la base des nombres de microorganismes comptés après une incubation réelle de 24 heures.

Comme il est possible de le constater, on observe une corrélation très forte entre la mesure réelle du nombre de microorganismes à 48 heures et l'estimation de ce même nombre, ces deux nombres différant de moins de 0.5 log.

Il est ainsi possible à l'aide l'estimation selon l'invention d'obtenir un résultat fiable à 24 heures de ce que sera le nombre de microorganismes à 48 heures. Ainsi, 24 heures à l'avance, il est possible de prendre les mesures adéquates dans le cas où le nombre estimé à 48 heures serait non-conforme aux exigences de sécurité alimentaire.

Bien que la méthode NPP ait été décrite pour réaliser le comptage, d'autres méthodes peuvent être utilisées. La méthode NPP a cependant l'avantage de pouvoir être automatisée, seule la préparation de l'échantillon biologique étant alors réalisée éventuellement de manière manuelle. Bien entendu, l'estimation du nombre de microorganismes selon l'invention peut également être réalisée en fonction d'un comptage manuel de colonies présentes sur une boite de Pétri après une durée d'incubation donnée.

Bien qu'il ait été décrit une application au dénombrement de microorganismes dans un produit alimentaire, notamment les viandes crues, d'autres produits peuvent être testés, comme par exemple les végétaux, les poissons ou tout autre produit à faible dure de vie.

## Revendications

1. Procédé de dénombrement de microorganismes présents dans un échantillon biologique en contact avec un milieu de culture adapté à la croissance desdits microorganismes, *caractérise* **en ce qu'**il comprend les étapes consistant à :

   - déterminer à un stade antérieur le nombre $N_{SA}$ de microorganismes présents dans l'échantillon
   - calculer (20) le nombre $N_{SU}$ de microorganismes présents à un stade ultérieur en fonction du nombre $N_{SA}$, le calcul se fondant sur un modèle de croissance des microorganismes dans le milieu de culture selon la relation :

$$\log(N_{SU}) = \alpha \times \log(N_{SA}) - \beta \times \log(C_{SA}) + \gamma$$

   où log est le logarithme décimal, $N_{SU}$ est le nombre de microorganismes calculé, $N_{SA}$ est le nombre de microorganismes au stade antérieur, $C_{SA}$ le nombre de microorganismes au stade antérieur divisé par le volume de l'échantillon, et $\alpha$, $\beta$ et $\gamma$ sont des paramètres prédéterminés dépendants des microorganismes, du milieu de culture et de la durée séparant le stade ultérieur du stade antérieur, $\alpha$ et $\beta$ étant positifs.

2. Procédé selon la revendication 1, *caractérisé* **en ce qu'**il comporte la détermination du nombre de microorganismes au stade antérieur (12-18), ladite estimation au stade antérieure comportant :

   ▪ la division (12) de l'échantillon entre plusieurs ensembles identiques de volume identique ou d'au moins deux volumes différents selon un rapport de proportionnalité prédéterminé ;
   ▪ l'application (14) de conditions de culture auxdits volumes, notamment une incubation à une température prédéterminée pendant une durée prédéterminée, afin de faire croitre le nombre de microorganismes dans ces volumes ;
   ▪ la détection (16) de la présence ou de l'absence de microorganismes dans les volumes après l'application des conditions de culture ; et
   ▪ le calcul (18) du nombre de microorganismes dans l'échantillon par la méthode du Nombre le Plus Probable.

3. Procédé selon la revendication 1 ou 2, *caractérisé* **en ce qu'**il comporte une étape de préparation (10) de l'échantillon biologique à partir d'une portion de produit alimentaire.

4. Procédé selon l'une quelconque des revendications précédentes, *caractérisé* **en ce que** le stade antérieur correspond à environ 24 heures après la préparation de l'échantillon, et **en ce que** le stade ultérieur correspond à environ 48 heures après la préparation de l'échantillon.

5. Dispositif pour le dénombrement de microorganismes présents dans un échantillon biologique en contact avec un milieu de culture adapté à la croissance desdits microorganismes, comportant des moyens formant réceptacle pour échantillon, et des moyens de mesure ou d'estimation du nombre de microorganismes présents dans un échantillon disposé dans le réceptacle pour échantillon, *caractérisé* **en ce qu'**il comporte en outre des moyens de calcul du nombre de microorganismes présents dans l'échantillon en fonction du nombre de microorganismes à un stade antérieur mesuré ou estimé par les moyens de mesure ou d'estimation, en se fondant sur un profil de croissance des microorganismes selon la relation :

$$\log(N_{SU}) = \alpha \times \log(N_{SA}) - \beta \times \log(C_{SA}) + \gamma$$

où log est le logarithme décimal, $N_{SU}$ est le nombre de microorganismes calculé, $N_{SA}$ est le nombre de microorganismes au stade antérieur mesuré ou estimé, $C_{SA}$ le nombre de microorganismes au stade antérieur divisé par le volume de l'échantillon, et $\alpha$, $\beta$ et $\gamma$ sont des paramètres prédéterminés dépendants de l'échantillon, du milieu de culture et de la durée séparant le stade ultérieur du stade antérieur, $\alpha$ et $\beta$ étant positifs.

**6.** Produit programme d'ordinateur comprenant des instructions, stockées sur un support d'enregistrement lisible par ordinateur, pour mettre en oeuvre le procédé de la revendication 1 lorsque le programme est exécuté par un ordinateur.

**Patentansprüche**

**1.** Verfahren zum Zählen von Mikroorganismen, die in einer biologischen Probe vorhanden sind, die mit einem Kulturmedium in Kontakt ist, das zum Wachstum der Mikroorganismen angepasst ist, **dadurch gekennzeichnet, dass** es die Schritte umfasst, die darin bestehen:

- in einem früheren Stadium die Anzahl $N_{SA}$ an in der Probe vorhandenen Mikroorganismen zu bestimmen,
- die Anzahl $N_{Su}$ an in einem späteren Stadium vorhandenen Mikroorganismen in Abhängigkeit von der Anzahl $N_{SA}$ zu berechnen (20), wobei die Berechnung auf einem Wachstumsmodell der Mikroorganismen im Kulturmedium nach dem Verhältnis basiert:

$$\log(N_{SU}) = \alpha \times \log(N_{SA}) - \beta \times \log(C_{SA}) + \gamma$$

worin log der Zehnerlogarithmus ist, $N_{SU}$ die berechnete Anzahl an Mikroorganismen ist, $N_{SA}$ die Anzahl an Mikroorganismen im früheren Stadium ist, $C_{SA}$ die Anzahl an Mikroorganismen im früheren Stadium dividiert durch das Volumen der Probe ist, und $\alpha$, $\beta$ und $\gamma$ vorbestimmte Parameter sind, die von den Mikroorganismen, dem Kulturmedium und der Dauer abhängen, die das spätere Stadium vom früheren Stadium trennt, wobei $\alpha$ und $\beta$ positiv sind.

**2.** Verfahren nach Anspruch 1, **dadurch gekennzeichnet, dass** es die Bestimmung der Anzahl an Mikroorganismen im früheren Stadium (12 - 18) umfasst, wobei die Schätzung im früheren Stadium umfasst:

■ Aufteilen (12) der Probe auf mehrere identische Einheiten mit gleichem Volumen oder zumindest zwei unterschiedlichen Volumina nach einem vorbestimmten Proportionalitätsverhältnis;
■ Anwenden (14) von Kulturbedingungen auf die Volumina, insbesondere eine Inkubation bei einer vorbestimmten Temperatur während einer vorbestimmten Dauer, um die Anzahl an Mikroorganismen in diesen Volumina zunehmen zu lassen;
■ Detektieren (16) des Vorhandenseins oder Nichtvorhandenseins von Mikroorganismen in den Volumina nach dem Anwenden der Kulturbedingungen; und
■ Berechnen (18) der Anzahl an Mikroorganismen in der Probe nach dem Verfahren der wahrscheinlichsten Anzahl.

**3.** Verfahren nach Anspruch 1 oder 2, **dadurch gekennzeichnet, dass** es einen Schritt der Vorbereitung (10) der biologischen Probe aus einem Teil eines Nahrungsmittelprodukts umfasst.

**4.** Verfahren nach einem der vorhergehenden Ansprüche, **dadurch gekennzeichnet, dass** das frühere Stadium ungefähr 24 Stunden nach der Vorbereitung der Probe entspricht, und dass das spätere Stadium ungefähr 48 Stunden nach der Vorbereitung der Probe entspricht.

**5.** Vorrichtung zum Zählen von Mikroorganismen, die in einer biologischen Probe vorhanden sind, die mit einem Kulturmedium in Kontakt ist, das zum Wachstum der Mikroorganismen angepasst ist, Einrichtungen, die ein Probengefäß bilden, und Mess- oder Schätzeinrichtungen für die Anzahl an Mikroorganismen umfassend, die in einer Probe vorhanden sind, die im Probengefäß angeordnet sind, **dadurch gekennzeichnet, dass** sie außerdem Einrichtungen zum Berechnen der Anzahl an in der Probe vorhandenen Mikroorganismen in Abhängigkeit von der Anzahl von Mikroorganismen in einem früheren Stadium umfasst, die durch die Mess- oder Schätzeinrichtungen basierend auf einem Wachstumsprofil der Mikroorganismen nach folgendem Verhältnis gemessen oder geschätzt wurden:

$$\log(N_{SU}) = \alpha \times \log(N_{SA}) - \beta \times \log(C_{SA}) + \gamma$$

worin log der Zehnerlogarithmus ist, $N_{SU}$ die berechnete Anzahl an Mikroorganismen ist, $N_{SA}$ die gemessene oder geschätzte Anzahl an Mikroorganismen im früheren Stadium ist, $C_{SA}$ die Anzahl an Mikroorganismen im früheren Stadium dividiert durch das Volumen der Probe ist, und $\alpha$, $\beta$ und $\gamma$ vorbestimmte Parameter sind, die von der Probe, dem Kulturmedium und der Dauer abhängen, die das spätere Stadium vom früheren Stadium trennt, wobei $\alpha$ und $\beta$ positiv sind.

**6.** Computerprogrammprodukt, das Befehle umfasst, die auf einem computerlesbaren Aufzeichnungsträger gespeichert sind, um das Verfahren nach Anspruch 1 umzusetzen, wenn das Programm durch einen Computer ausgeführt wird.

**Claims**

**1.** A method for counting microorganisms present in a biological sample in contact with a culture medium adapted to the growth of said microorganisms, **characterized in that** it comprises the steps of:

- determining at a prior stage number $N_{SA}$ of microorganisms present in the sample
- calculating (20) number $N_{SU}$ of microorganisms present in the sample according to number $N_{SA}$, the calculation being based on a model of microorganism growth in the culture medium according to the following relation:

$$\log(N_{SU}) = \alpha \times \log(N_{SA}) - \beta \times \log(C_{SA}) + \gamma$$

where log is the decimal logarithm, $N_{SU}$ is the calculated number of microorganisms, $N_{SA}$ is the number of microorganisms at the prior stage, $C_{SA}$ is the number of microorganisms at the prior stage divided by the volume of the sample, and $\alpha$, $\beta$, and $\gamma$ are determined parameters depending on the microorganisms, on the culture medium, and on the time period separating the subsequent stage from the prior stage, $\alpha$ and $\beta$ being positive.

**2.** The method of claim 1, **characterized in that** it comprises determining the number of microorganisms at the prior stage (12-18), said estimate at the prior stage comprising:

- dividing (12) the sample into several identical assemblies of identical volume or of at least two different volumes, according to a predetermined proportionality factor;
- applying (14) culture conditions to said volumes, and especially an incubation at a predetermined temperature for a predetermined time period, to have the number of microorganisms grow in these volumes;
- detecting (16) the presence or the absence of microorganisms in the volumes after having applied the culture conditions; and
- calculating (18) the number of microorganisms in the sample by the most probable number method.

**3.** The method of claim 1 or 2, **characterized in that** it comprises a step of preparation (10) of the biological sample from a portion of food product.

4. The method of any of the foregoing claims, ***characterized in that*** the prior stage corresponds to approximately 24 hours after the preparation of the sample and **in that** the subsequent stage corresponds to approximately 48 hours after the preparation of the sample.

5. A device for counting microorganisms present in a biological sample in contact with a culture medium adapted to the growth of said microorganisms, comprising means forming a sample receiver, and means for measuring or estimating the number of microorganisms present in a sample arranged in the sample receiver, ***characterized in that*** it further comprises means for calculating the number of microorganisms present in the sample according to the number of microorganisms at a prior stage measured or estimated by the measurement or estimating means, based on a model of growth of the microorganisms according to the following relation:

$$\log(N_{SU}) = \alpha \times \log(N_{SA}) - \beta \times \log(C_{SA}) + \gamma$$

where log is the decimal logarithm, $N_{SU}$ is the calculated number of microorganisms, $N_{SA}$ is the measured or estimated number of microorganisms at the prior stage, $C_{SA}$ is the number of microorganisms at the prior stage divided by the volume of the sample, and $\alpha$, $\beta$ and $\gamma$ are determined parameters depending on the microorganisms, on the culture medium, and on the time period separating the subsequent stage from the prior stage, $\alpha$ and $\beta$ being positive.

6. A computer program product comprising instructions, stored on a computer-readable recording support, for implementing the method of claim 1 when the program is executed by a computer.

Fig. 1

Fig. 2

**EP 2 547 783 B1**

## RÉFÉRENCES CITÉES DANS LA DESCRIPTION

**Documents brevets cités dans la description**

- EP 1105457 A